# EUROPEAN PATENT SPECIFICATION

(11) **EP 3 124 118 B1**
(45) Date of publication and mention of the grant of the patent: **28.02.2018**
(21) Application number: 16170761.7
(22) Date of filing: 23.05.2016
(51) Int. Cl.: B01L 7/00, B01L 3/00, C12Q 1/68

(54) **MICROCHANNEL APPARATUS AND PCR METHOD**
MIKROKANALVORRICHTUNG UND PCR-VERFAHREN
DISPOSITIF À MICROCANAL ET PROCÉDÉ PCR

(30) Priority: 28.07.2015 JP 2015148540
(43) Date of publication of application: 01.02.2017
(73) Proprietor: Panasonic Intellectual Property Management Co., Ltd., Osaka-shi, Osaka 540-6207 (JP)
(72) Inventor: Hishida, Mitsuoki, Osaka, 540-6207 (JP)
(74) Representative: Grünecker Patent- und Rechtsanwälte PartG mbB

(56) References cited:
- US-A1- 2007 292 941
- US-A1- 2009 215 023
- US-A1- 2010 266 456
- US-A1- 2013 263 940

## Description

### BACKGROUND

### 1. Technical Field

The present disclosure relates to a microchannel chip, a polymerase chain reaction (PCR) method, and a heating/cooling control apparatus, each of which is used in performing a PCR process on a treatment target liquid.

### 2. Description of the Related Art

PTL1 discloses a heating-reaction use microchip that includes a reaction region for various reactions to occur, and a heating control field that has a heat generating element and a liquid and controls heating of the reaction region.

### Citation List

### Patent Literature

PTL 1: Unexamined Japanese Patent Publication No. 2013-85530

With the microchip according to PTL1, for the purpose of conducting heat to the reaction region, heat of the heat generating element is conducted to the reaction region through the liquid. It is not considered as to the responsiveness of the heat conduction through the liquid. US2009/0215023, US2013/0263940 and US2007/0292941 also disclose heated microchannel PCR devices.

### SUMMARY

The invention relates to a PCR method and microchannel apparatus as claimed in claims 1 and 8, with which the responsiveness of the temperature of a treatment target liquid undergoing a PCR process can be improved using a temperature regulator.

In one general aspect, the techniques disclosed here feature a polymerase chain reaction (PCR) method for performing a PCR process on a treatment target liquid using a microchannel apparatus including a microchannel chip and a heating/cooling control apparatus, the PCR method including:
(a) preparing the microchannel apparatus including the microchannel chip and the heating/cooling control apparatus;
   the microchannel chip including:
   a first substrate layer comprising an introducing channel and a discharging channel; and
   a second substrate layer that is disposed on the first substrate layer and comprises a second substrate layer body and a metal film, the second substrate layer body comprising a microchannel connected to the introducing channel and the discharging channel, the microchannel being filled with the treatment target liquid, the metal film structuring an upper surface of the microchannel,
   the heating/cooling control apparatus including:
      a temperature regulator that is disposed so as to be in contact with the metal film of the second substrate layer and is capable of heating or cooling the metal film;
      a power source that applies voltage to the temperature regulator; and
      a heating/cooling controller that controls the voltage applied from the power source to the temperature regulator;
(b) controlling, with the heating/cooling control apparatus, the voltage applied from the power source to the temperature regulator so as to change a temperature of the treatment target liquid in the microchannel, thereby achieving the PCR process, wherein an end of the metal film of the second substrate layer in the microchannel chip is buried in the second substrate layer body.

According to the aspect of the present disclosure, diffusion of heat of the temperature regulator can be reduced, and the temperature regulator can improve the responsiveness of the temperature of the treatment target liquid.

Additional benefits and advantages of the disclosed embodiments will become apparent from the specification and drawings. The benefits and/or advantages may be individually obtained by the various embodiments and features of the specification and drawings, which need not all be provided in order to obtain one or more of such benefits and/or advantages.

It should be noted that general or specific embodiments may require a system, a method, an integrated circuit, a computer program, a storage medium, or any selective combination thereof.

### BRIEF DESCRIPTION OF THE DRAWINGS

FIG. 1A is a perspective view showing the overall structure of a microchannel apparatus according to one exemplary embodiment;
FIG. 1B is a perspective view, as seen from above, of a microchannel portion of the microchannel apparatus according to the exemplary embodiment;
FIG. 1C is a perspective view, as seen from below, of the microchannel portion of the microchannel apparatus according to the exemplary embodiment;
FIG. 2A is a vertical cross-sectional view of microchannels and others in the microchannel apparatus according to the exemplary embodiment before placement of a temperature regulator;
FIG. 2B is a partially perspective plan view of the microchannels and others in the microchannel apparatus shown in FIG. 2A before placement of the temperature regulator;
FIG. 2C is a vertical cross-sectional view of the microchannels and others in the microchannel apparatus shown in FIG. 2A after placement of the temperature regulator; FIG. 2D is a vertical cross-sectional view of microchannels and others in a microchannel apparatus according to Variation of the exemplary embodiment after placement of the temperature regulator;
FIG. 2E is a partially perspective plan view of the microchannels and others in the microchannel apparatus according to Variation of the exemplary embodiment shown in FIG. 2D after placement of the temperature regulator;
FIG. 3A is a vertical cross-sectional view of the microchannels for describing a process flow with the microchannel apparatus;
FIG. 3B is a vertical cross-sectional view of the microchannels and others for describing the process flow with the microchannel apparatus;
FIG. 3C is a vertical cross-sectional view of the microchannels and others for describing the process flow with the microchannel apparatus;
FIG. 3D is a vertical cross-sectional view of the microchannels and others for describing the process flow with the microchannel apparatus;
FIG. 3E is a vertical cross-sectional view of the microchannels and others for describing the process flow with the microchannel apparatus;
FIG. 3F is a flowchart showing the process flow with the microchannel apparatus;
FIG. 4A is a plan view showing disposition of a metal film relative to the microchannel in which the metal film and a substrate layer body are each hatched so as to be distinguishable from each other;
FIG. 4B is a plan view of a metal film according to Variation in which the metal film and a substrate layer body are each hatched so as to be distinguishable from each other; FIG. 4C is a cross-sectional view taken along line 4C-4C in FIG. 4B;
FIG. 5A is an enlarged cross-sectional view for describing the relationship between the temperature regulator and the metal film;
FIG. 5B is an enlarged cross-sectional view of a metal film according to Variation;
FIG. 5C is a plan view of the metal film according to Variation shown in FIG. 5B in which the metal film and a substrate layer body are each hatched so as to be distinguishable from each other; Note that FIG. 5 is not according to the presently claimed invention.
FIG. 6A is a vertical cross-sectional view for describing a process of molding the microchannel and the metal film;
FIG. 6B is a vertical cross-sectional view for describing a process of molding the microchannel and the metal film;
FIG. 6C is a vertical cross-sectional view for describing a process of molding the microchannel and the metal film;
FIG. 6D is a vertical cross-sectional view for describing a process of molding the microchannel and the metal film; and
FIG. 6E is a partially enlarged vertical cross-sectional view for describing a process of molding the microchannel and the metal film.

### DETAILED DESCRIPTION

In the following, a detailed description will be given of an exemplary embodiment according to the present disclosure with reference to the drawings.

Prior to the detailed description of the exemplary embodiment according to the present disclosure with reference to the drawings, various aspects of the present disclosure are described hereinafter.

A first aspect of the present disclosure provides a polymerase chain reaction (PCR) method for performing a PCR process on a treatment target liquid using a microchannel apparatus including a microchannel chip and a heating/cooling control apparatus, the PCR method including:
(a) preparing the microchannel apparatus including the microchannel chip and the heating/cooling control apparatus;
   the microchannel chip including:
   a first substrate layer that has an introducing channel and a discharging channel; and
   a second substrate layer that is disposed on the first substrate layer and has a second substrate layer body and a metal film, the second substrate layer body having a microchannel connected to the introducing channel and the discharging channel, the microchannel being filled with the treatment target liquid, the metal film structuring an upper surface of the microchannel,
   the heating/cooling control apparatus including:
      a temperature regulator that is disposed so as to be in contact with the metal film of the second substrate layer and capable of changing a temperature of the metal film;
      a power source that supplies voltage to the temperature regulator; and
      a heating/cooling control apparatus that controls the voltage applied from the power source to the temperature regulator;
(b) controlling, by the heating/cooling controller, the voltage applied from the power source to the temperature regulator so as to change a temperature of the temperature regulator; and
(c) allowing heat of the temperature regulator to be conducted to the liquid in the microchannel via the metal film to change a temperature of the liquid, thereby achieving the PCR process, wherein an end of the metal film of the second substrate layer in the microchannel chip is buried in the second substrate layer body.

According to the aspect of the present disclosure, diffusion of heat of the temperature regulator can be reduced, and the temperature regulator can improve the responsiveness of the temperature of the treatment target liquid.

A second aspect of the present disclosure provides the PCR method according to the first aspect, wherein
the microchannel chip further includes:
an introduction-side valve that opens or closes the introducing channel; and
a discharge-side valve that opens or closes the discharging channel,
the heating/cooling control apparatus further includes
a valve controller that controls the introduction-side valve and the discharge-side valve to open or close, the PCR method further including, prior to the step (b),
(c) closing the introduction-side valve and the discharge-side valve under control of the valve controller in a state where the microchannel is filled with the liquid.

According to the aspect of the present disclosure, the structure that prevents leakage of the liquid during the PCR process is advantageously obtained.

A third aspect of the present disclosure provides the PCR method according to one of the first and second aspects, wherein the second substrate layer body is made of dimethylpolysiloxane, and the metal film is made of aluminum.

According to the aspect of the present disclosure, use of aluminum being higher than dimethylpolysiloxane in thermal conductivity advantageously allows the temperature regulator to quickly heat or cool.

A fourth aspect of the present disclosure provides the PCR method according to any one of the first to third aspects, wherein the temperature regulator is a Peltier element.

According to the aspect of the present disclosure, the Peltier element can raise or lower the temperature to any value and conduct the heat to the metal film, thereby achieving the PCR process.

A fifth aspect of the present disclosure provides the PCR method according to any one of the first to fourth aspects, wherein the metal film includes a plurality of metal films that are spaced apart from each other and arranged along the microchannel.

According to the aspect of the present disclosure, even when the surface of the temperature regulator is uneven or distorted, the temperature regulator can be efficiently brought into contact with the metal film. Thus, the efficient heating and cooling process is realized.

A sixth aspect of the present disclosure provides the PCR method according to the second aspect, further including, after the step (b), (d) opening the introduction-side valve and the discharge-side valve so as to discharge, from the discharge-side valve, the liquid having undergone the PCR process.

According to the aspect of the present disclosure, the structure that prevents leakage of the liquid during the PCR process is advantageously obtained, and the liquid can be taken out at any timing.

A seventh aspect of the present disclosure provides the PCR method according to any one of the first to sixth aspects, wherein, in the step (b), the heating/cooling controller controls the change in the temperature of the temperature regulator in accordance with a type of the liquid, based on relationship information among the type of the liquid, the voltage to the temperature regulator, a period of applying the voltage, and the temperature.

According to the aspect of the present disclosure, the heating or cooling period can be efficiently controlled.

An eighth aspect of the present disclosure provides a microchannel apparatus according to claim 8.

According to the aspect of the present disclosure, diffusion of heat of the temperature regulator can be reduced, and the temperature regulator can improve the responsiveness of the temperature of the treatment target liquid.

A ninth aspect of the present disclosure provides the microchannel chip according to the eighth aspect, wherein
the microchannel chip includes:
an introduction-side valve that opens or closes the introducing channel; and
a discharge-side valve that opens or closes the discharging channel.

According to the aspect of the present disclosure, the flow of the liquid can be blocked for any period by causing the introduction-side valve and the discharge-side valve to be pushed.

A tenth aspect of the present disclosure provides the microchannel chip according to one of the eighth and ninth aspects, wherein
the second substrate layer body is made of dimethylpolysiloxane, and
the metal film is made of aluminum.

According to the aspect of the present disclosure, the heat by the temperature regulator is not easily conducted to the second substrate layer body and tends to stay at the metal film.

According to the invention, an end of the metal film of the second substrate layer in the microchannel chip is buried in the second substrate layer body.

According to the aspect of the present disclosure, since the end of the metal film is buried in the second substrate layer body, the effect of preventing leakage of the liquid can be achieved.

### EXEMPLARY EMBODIMENT

In the following, a description will be given of one exemplary embodiment with reference to the drawings.

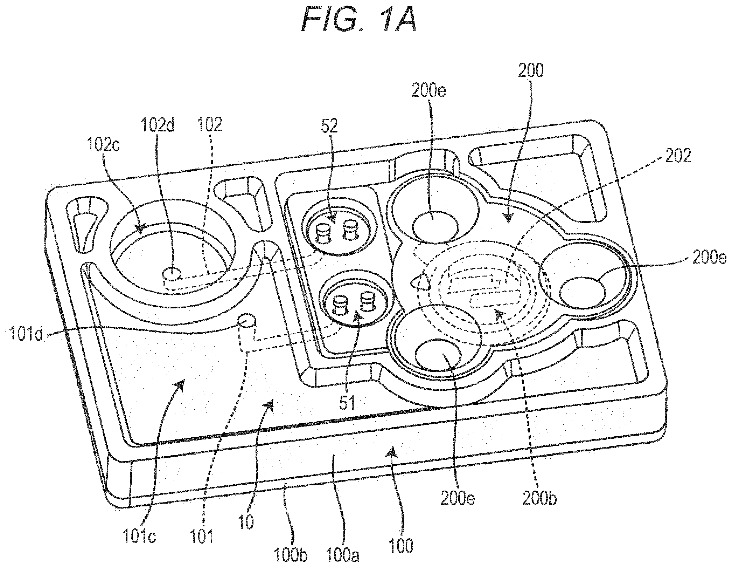

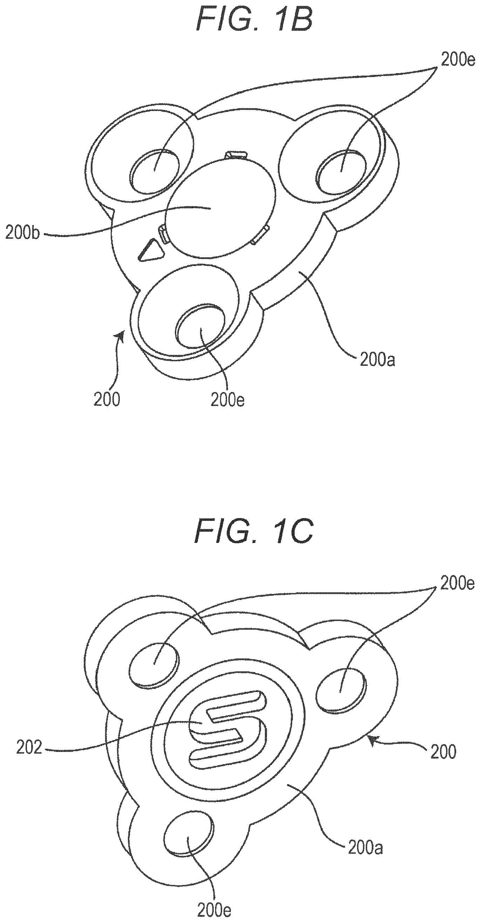

FIG. 1A is a perspective view of the overall structure of microchannel system 1, which is a microchannel apparatus according to one exemplary embodiment of the present disclosure. FIGS. 1B and 1C are perspective views, as seen from above and below, respectively, of a microchannel portion of the microchannel apparatus according to the present exemplary embodiment. Microchannel system 1 shown in FIGS. 1A to 1C includes microchannel chip 10 and heating/cooling control apparatus 20 being an exemplary heating/cooling control apparatus. Microchannel chip 10 includes first substrate layer 100 and second substrate layer 200. Heating/cooling control apparatus 20 includes temperature regulator 11, power source 12, and heating/cooling controller 13.

Microchannel system 1 amplifies DNA in treatment target liquid 50 in microchannel chip 10 through the polymerase chain reaction (PCR) method. Specifically, microchannel system 1 controls temperatures of treatment target liquid 50 in microchannel chip 10 through use of temperature regulator 11, power source 12, and heating/cooling controller 13. Treatment target liquid 50 contains a gene (i.e., DNA). Treatment target liquid 50 may be referred to as "Analyte".

### (Microchannel chip 10)

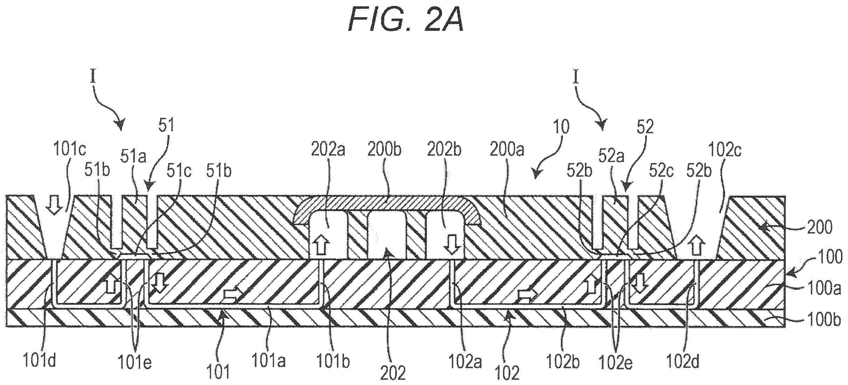

FIGS. 2A to 2C are vertical cross-sectional views and a plan view of microchannel chip 10.

Microchannel chip 10 includes first substrate layer 100 and second substrate layer 200.

### (First substrate layer 100)

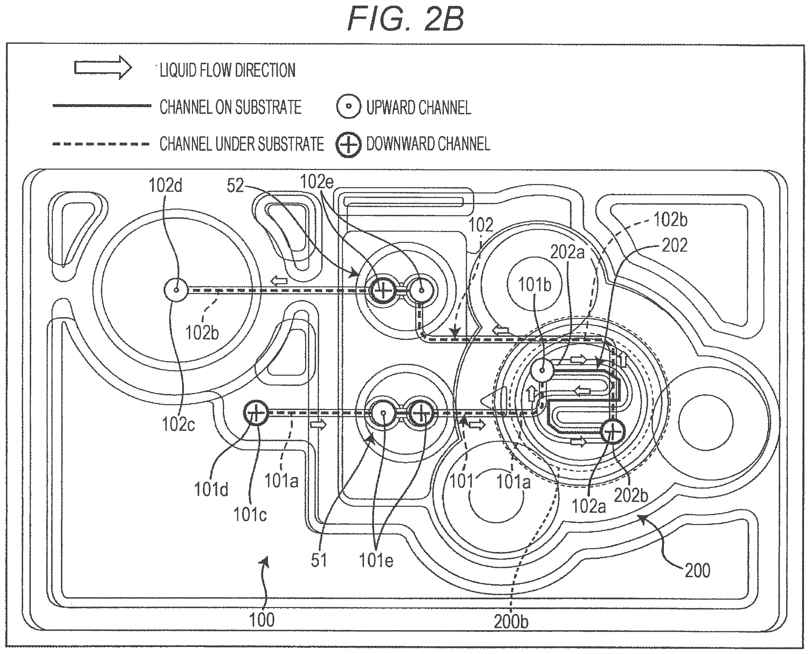

As shown in FIGS. 2A and 2B, first substrate layer 100 is structured by first substrate layer body 100a that includes introducing channel 101 and discharging channel 102, and back plate 100b that is fixed to the lower surface of first substrate layer 100. Note that, while FIG. 1A shows the bottom surface of channels 101, 102 of first substrate layer 100 being structured by back plate 100b, back plate 100b can be dispensed with if introducing channel 101 and discharging channel 102 are structured inside first substrate layer body 100a.

Exemplary materials of first substrate layer body 100a include dimethylpolysiloxane (PDMS), polycarbonate, acrylic resin (polymethyl methacrylate (PMMA)), and silicone. Exemplary materials of back plate 100b include dimethylpolysiloxane, polycarbonate, acrylic resin, silicone, and glass.

Introducing channel 101 includes first channel 101a, second channel 101b, and fifth channel 101d.

First channel 101a extends in parallel to the surface of first substrate layer 100 via introduction-side valve 51 which will be described later.

Second channel 101b is connected to the downstream end side of first channel 101a, and extends substantially perpendicularly to the surface of first substrate layer 100 (that is, toward second substrate layer 200), to penetrate through first substrate layer 100 in the thickness direction.

Fifth channel 101d is connected to the upstream end side of first channel 101a, and extends substantially perpendicularly to the surface of first substrate layer 100 (that is, toward second substrate layer 200), to penetrate through first substrate layer 100 in the thickness direction.

Discharging channel 102 includes third channel 102a, fourth channel 102b, and sixth channel 102d.

Third channel 102a extends in parallel to the surface of first substrate layer 100 via introduction-side valve 51 which will be described later.

Fourth channel 102b extends substantially perpendicularly to the surface of first substrate layer 100, to penetrate through first substrate layer 100 in the thickness direction.

Fifth channel 101d is connected to the upstream end side of first channel 101a, and extends substantially perpendicularly to the surface of first substrate layer 100, to penetrate through first substrate layer 100 in the thickness direction.

### (Second substrate layer 200)

As shown in FIGS. 2A and 2B, second substrate layer 200 is disposed on first substrate layer 100. Second substrate layer 200 includes at least second substrate layer body 200a that has microchannel 202 at its inner central portion, and metal film 200b that is fixed to the upper portion of the central portion of substrate layer body 200a so as to structure the upper surface of microchannel 202.

The circumference of metal film 200b enters inside substrate layer body 200a, as if it is pressed down by an In such a structure, metal film 200b having high thermal conductivity is partially covered with the material of substrate layer body 200a. This increases the contact area between metal film 200b and substrate layer body 200a, minimizing leakage of liquid 50. Note that, FIG. 1A and others show screw holes 200e for fixing second substrate layer 200 to first substrate layer 100.

Metal film 200b includes the outer surface that is brought into direct contact with temperature regulator 11, and the inner surface that is brought into direct contact with treatment target liquid 50. An exemplary material of metal film 200b is aluminum having high thermal conductivity. When microchannel 202 is filled with liquid 50, liquid 50 is in direct contact with metal film 200b being the upper surface of microchannel 202. Thus, as will be described later, heat can be conducted from metal film 200b to liquid 50.

An exemplary material of second substrate layer 200 is silicone-based resin. A specific example of silicone-based resin is dimethylpolysiloxane. That is, second substrate layer 200 being made of a low-thermal-conductivity material reduces diffusion of heat, which is conducted from temperature regulator 11 to metal film 200b, to substrate layer body 200a. Further, metal film 200b, which is the upper surface of second substrate layer 200 brought into direct contact with treatment target liquid 50, being made of high-thermal-conductivity metal allows temperature regulator 11 to control the temperature of treatment target liquid 50 via metal film 200b with ease.

Second substrate layer 200 may further integrally include introduction-side valve 51, discharge-side valve 52, introducing opening 101c, and discharging opening 102c.

Introduction-side valve 51 is integrally formed with second substrate layer 200 as a part of second substrate layer 200 around the intermediate portion of first channel 101a of introducing channel 101.

Introduction-side valve 51 is structured by valve body 51a, thin and elastically deformable supporters 51b that constantly apply biasing force to valve body 51a so as to be kept at open position I, and recess 51c that is formed at the bottom surface of valve body 51a. Opening ends of a pair of upward channels 101e at the intermediate portion of first channel 101a of introducing channel 101 face recess 51c. Accordingly, when valve body 51a is at the upper end position, i.e., open position I, by the biasing force of supporters 51b, upward channels 101e communicate with each other via recess 51c. Accordingly, for example, treatment target liquid 50 is allowed to be introduced from introducing opening 101c to microchannel 202 via first channel 101a and second channel 101b of introducing channel 101. On the other hand, when valve body 51a is lowered against the biasing force of supporters 51b and positioned at close position II, the pair of upward channels 101e is closed by the elastically deformed bottom surface of recess 51c; first channel 101a is closed at the intermediate portion; and treatment target liquid 50 can be packed and retained in introducing channel 101 and microchannel 202. Opening and closing operations of introduction-side valve 51 in such a manner enables control over introduction and packing and retaining of liquid 50 flowing through first channel 101a.

Discharge-side valve 52 is integrally formed with second substrate layer 200 as a part of second substrate layer 200 around the intermediate portion of fourth channel 102b of discharging channel 102.

Similarly to introduction-side valve 51, discharge-side valve 52 is structured by valve body 52a, thin and elastically deformable supporters 52b that constantly apply biasing force to valve body 52a so as to be kept at open position I, and recess 52c that is formed at the bottom surface of valve body 52a. Opening ends of a pair of upward channels 102e at the intermediate portion of fourth channel 102b of discharging channel 102 face recess 52c. Accordingly, when valve body 52a is at the upper end position, i.e., open position I, by the biasing force of supporters 52b, upward channels 102e communicate with each other via recess 52c. Accordingly, for example, treatment target liquid 50 is allowed to be discharged from microchannel 202 through discharging opening 102c via third channel 102a and fourth channel 102b of discharging channel 102. On the other hand, when valve body 52a is lowered against the biasing force of supporters 52b and positioned at close position II, the pair of upward channels 102e is closed by the elastically deformed bottom surface of recess 52c; fourth channel 102b is closed at the intermediate portion; and treatment target liquid 50 can be packed and retained in microchannel 202 and discharging channel 102. Opening and closing operations of discharge-side valve 52 in such a manner enables control over discharge of the liquid flowing through fourth channel 102b.

Note that, for example, introduction-side valve 51 and discharge-side valve 52 may be normally kept at open position I by the biasing force of supporters 51b, 52b. When introduction-side valve 51 and discharge-side valve 52 are to be closed, introduction-side valve 51 and discharge-side valve 52 may be pressed so as to position at close position II against the biasing force of supporters 51b, 52b.

Microchannel 202 includes first end 202a that is connected to the downstream end of introducing channel 101, and second end 202b that is connected to the upstream end of discharging channel 102. Microchannel 202 is formed to meander, for example, in an S-shaped manner, from first end 202a to second end 202b. At the upper end surface of microchannel 202, the inner surface of heating/cooling-purpose metal film 200b, which is fixed to the upper portion of the central portion of second substrate layer 200 and has a circular shape, for example, is exposed. In other words, microchannel 202 meanders in an S-shaped manner so that the liquid is brought into contact with the inner surface of metal film 200b to a maximum extent, and thus liquid 50 in microchannel 202 can be efficiently brought into contact with metal film 200b of a smaller area. Hence, from the downstream end of first channel 101a of introducing channel 101 at first substrate layer 100, treatment target liquid 50 is introduced into first end 202a of microchannel 202. The introduced treatment target liquid 50 meanderingly flows through microchannel 202 while being brought into contact with metal film 200b and thereby heated or cooled. Then, from second end 202b of microchannel 202, treatment target liquid 50 is discharged to the upstream end of discharging channel 102 at first substrate layer 100.

At the outer surface of metal film 200b structuring the upper surface of microchannel 202 at second substrate layer 200, temperature regulator 11 is disposed so as to be in contact therewith. By the outer surface of metal film 200b and temperature regulator 11 being in contact with each other, heat of temperature regulator 11 can be efficiently conducted to treatment target liquid 50 via metal film 200b at the upper surface of second substrate layer 200.

To the upstream end of introducing channel 101, introducing opening 101c is connected via introduction-side valve 51.

Introducing opening 101c is a recess having an inclined surface. Introducing opening 101c penetrates through second substrate layer 200 in the thickness direction, to be connected to the upstream end of fifth channel 101d. For example, treatment target liquid 50 is supplied to introducing opening 101c by a pump or a dropper. Then, treatment target liquid 50 enters first channel 101a from fifth channel 101d of introducing channel 101, and flows through first channel 101a to be introduced to microchannel 202 at second substrate layer 200 from second channel 101b.

Further, to the downstream end of discharging channel 102, discharging opening 102c is connected via discharge-side valve 52.

Discharging opening 102c is a recess having an inclined surface. Discharging opening 102c penetrates through second substrate layer 200 in the thickness direction, to be connected to the downstream end of sixth channel 102d. After a PCR process, treatment target liquid 50 is discharged from microchannel 202 at second substrate layer 200 to third channel 102a of discharging channel 102, and flows through fourth channel 102b, to be output from sixth channel 102d and discharging opening 102c to a DNA sensing apparatus (not shown), for example.

### (Heating/cooling control apparatus 20)

Heating/cooling control apparatus 20 includes housing 20a, temperature regulator 11 fixed to the central portion of the lower surface of housing 20a, power source 12, and heating/cooling controller 13. Heating/cooling control apparatus 20 may further include valve controller 104 on each of the opposite sides on lower surface of housing 20a.

Power source 12 and heating/cooling controller 13 may be disposed inside or outside housing 20a.

Temperature regulator 11 is fixed to the lower surface at the central portion of housing 20a of heating/cooling control apparatus 20. Valve controller 104 is disposed on each of the opposite sides of temperature regulator 11. Hence, disposition of housing 20a on microchannel chip 10 causes temperature regulator 11 to be placed on metal film 200b so as to be in contact therewith. By temperature regulator 11 heating or cooling metal film 200b executes a PCR process on liquid 50 retained in microchannel 202 of microchannel chip 10. Thus, DNA can be amplified.

As being placed on the upper surface of second substrate layer 200, temperature regulator 11 heats or cools treatment target liquid 50 in microchannel 202 of microchannel chip 10. Exemplary temperature regulator 11 is a Peltier element.

Temperature regulator 11 is electrically connected to heating/cooling controller 13 and power source 12 via electrical lines. Power source 12 applies voltage to temperature regulator 11.

Heating/cooling controller 13 refers, for example, to the relationship information among the type of liquid 50, a predetermined temperature, a period of applying the voltage, the voltage to temperature regulator 11 or the standard thereof, and controls the temperature of temperature regulator 11 by controlling the voltage application of power source 12. The predetermined relationship information or the standard is the relationship information or the standard for controlling temperatures in any known PCR method. As to any known PCR method, for example, see the document (Joan M. Henson et. al., "POLYMERASE CHAIN REACTION AND PLANT DISEASE DIAGNOSIS", Annu. Rev. Phytopathol, 1993, 31, pp. 81-109).

Valve controller 104 controls opening/closing operations of introduction-side valve 51 and discharge-side valve 52.

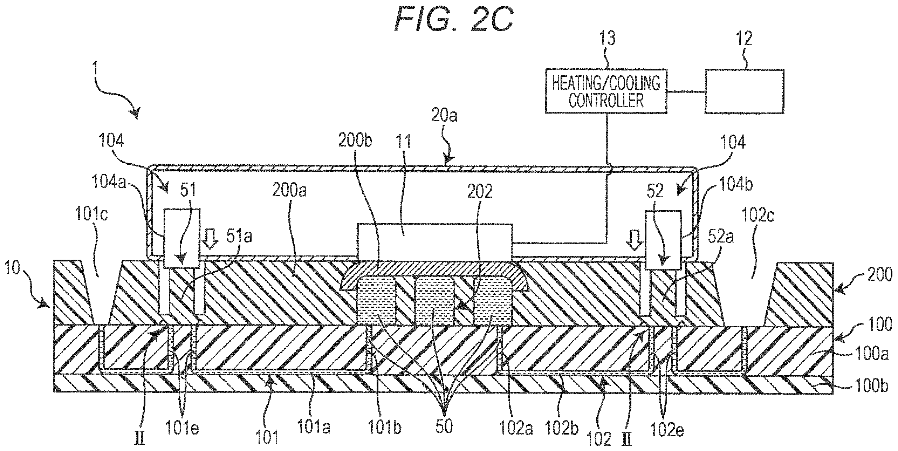

For example, as shown in FIG. 2C, exemplary valve controller 104 is structured by first projection 104a and second projection 104b that are fixed on the opposite sides of temperature regulator 11 at a lower portion of housing 20a of heating/cooling control apparatus 20 as being projecting downward. When temperature regulator 11 is placed together with housing 20a on metal film 200b of microchannel chip 10 so as to be in contact therewith, first projection 104a presses introduction-side valve 51 downward to cause elastic deformation thereby closing introduction-side valve 51, and second projection 104b presses discharge-side valve 52 downward to cause elastic deformation thereby closing discharge-side valve 52. That is, during the period in which temperature regulator 11 is placed on metal film 200b of microchannel chip 10, introduction-side valve 51 and discharge-side valve 52 are kept in the closed state by first projection 104a and second projection 104b (i.e., close position II). Conversely, during the period in which temperature regulator 11 is spaced apart above from metal film 200b of microchannel chip 10, introduction-side valve 51 and discharge-side valve 52 are kept in the open state by being released from the pressing of first projection 104a and second projection 104b (i.e., open position I). The raising/lowering operations of housing 20a integrally with temperature regulator 11 and valve controller 104 can be realized through use of any known raising/lowering apparatus such as a linear-motion mechanism made up of a motor and a ball screw shaft or an air cylinder.

Accordingly, for example, by temperature regulator 11 being placed on microchannel chip 10 after treatment target liquid 50 of at least a prescribed capacity is disposed in first channel 101a, microchannel 202, and fourth channel 102b, valve controller 104 simultaneously closes introduction-side valve 51 and discharge-side valve 52.

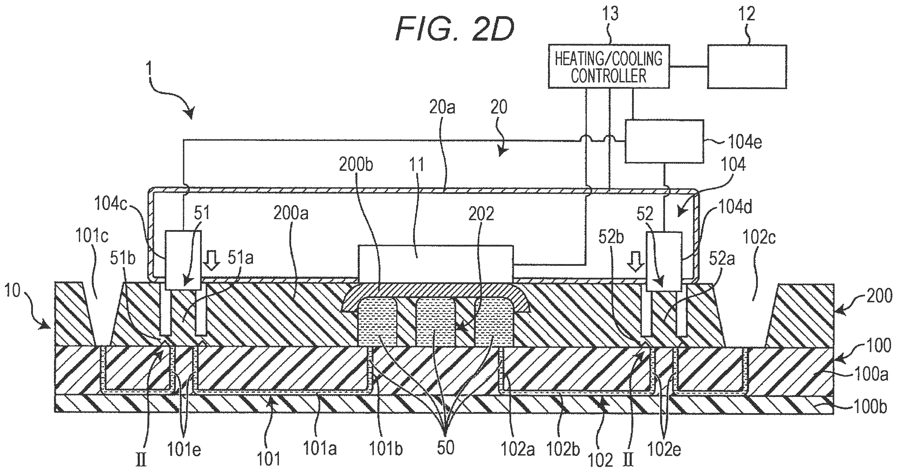

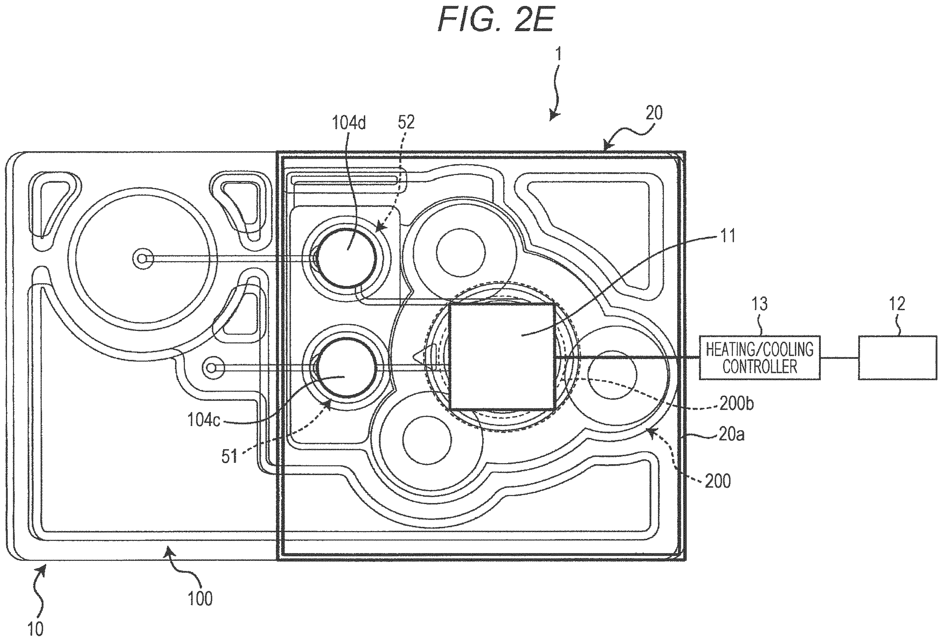

Further, valve controller 104 may be in other exemplary structure shown in FIGS. 2D and 2E, in place of that shown in FIG. 2C. This other exemplary valve controller 104 is structured by first and second actuators 104c, 104d made of shape memory alloy that is capable of vertically expanding and contracting, and actuator controller 104e that controls expansion and contraction of each of first and second actuators 104c, 104d. Use of shape memory alloy as first and second actuators 104c, 104d provides excellent responsiveness, and also advantageous in miniaturizing microchannel system 1 because great drive force is not required. When actuator controller 104e applies voltage or stops applying voltage to each shape memory alloy, first and second actuators 104c, 104d are driven or stopped, whereby expansion/contraction operations of each of first and second actuators 104c, 104d are controlled. The expansion/contraction operations of first and second actuators 104c, 104d control opening/closing operations of introduction-side valve 51 and discharge-side valve 52. For example, for closing introduction-side valve 51 and discharge-side valve 52, first and second actuators 104c, 104d are caused to expand so as to press downward introduction-side valve 51 and discharge-side valve 52 against the biasing force of supporters 51b, 52b. Then, introduction-side valve 51 and discharge-side valve 52 are deformed so as to lower from open position I to close position II against the biasing force of supporters 51b, 52b. Conversely, for opening introduction-side valve 51 and discharge-side valve 52, first and second actuators 104c, 104d are caused to contract so as to extinguish the pressing force on introduction-side valve 51 and discharge-side valve 52. Then, introduction-side valve 51 and discharge-side valve 52 are pushed upward by the biasing force of supporters 51b, 52b, and rise from close position II to open position I.

Heating/cooling controller 13 and actuator controller 104e may be implemented by one or more electronic circuit that includes a semiconductor apparatus, a semiconductor integrated circuit (i.e., IC), or an LSI (i.e., large scale integration). The LSI or the IC may be integrated on one chip, or may be structured by a combination of a plurality of chips. For example, functional blocks other than a memory element may be integrated on one chip.

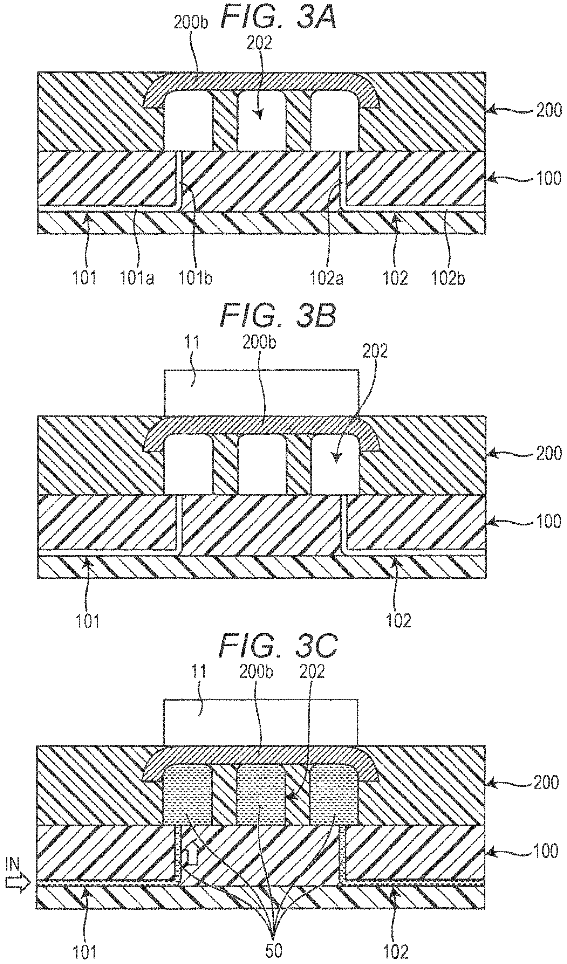

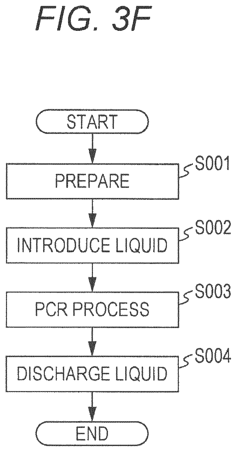

With reference to FIGS. 3A to 3F, a process flow with microchannel system 1 is described.

### (Step S001)

Firstly, microchannel system 1 is prepared. That is, under housing 20a of heating/cooling control apparatus 20, microchannel chip 10 shown in FIG. 3A is installed. Specifically, above the upper surface of second substrate layer 200 (that is, metal film 200b), temperature regulator 11 that is fixed to housing 20a of heating/cooling control apparatus 20 is disposed. Here, introduction-side valve 51 and discharge-side valve 52 are positioned at open position I.

Note that, when valve controller 104 is structured by first projection 104a and second projection 104b, as described above, in Step S001, temperature regulator 11 is positioned so as to be spaced apart above from the upper surface of second substrate layer 200 (i.e., metal film 200b).

Meanwhile, when valve controller 104 is structured by first and second actuators 104c, 104d and actuator controller 104e, as shown in FIG. 3B, temperature regulator 11 is placed on the upper surface of second substrate layer 200 (i.e., metal film 200b) together with housing 20a. Here, actuator controller 104e does not drive first and second actuators 104c, 104d and valves 51, 52 are kept at open position I.

### (Step S002)

Next, as shown in FIG. 3C, treatment target liquid 50 is introduced into introducing channel 101 from introducing opening 101c at first substrate layer 100.

For example, in the state where introduction-side valve 51 and discharge-side valve 52 are at open position I by being opened by valve controller 104, liquid 50 is introduced into introducing channel 101. When liquid 50 is packed from introducing channel 101 to discharging channel 102 via microchannel 202 and started to be discharged from discharging opening 102c at the downstream end of discharging channel 102, introduction-side valve 51 and discharge-side valve 52 are closed. Thus, treatment target liquid 50 is packed in microchannel 202. Note that, valves 51, 52 may be controlled to close through use of a sensor (not shown) disposed at discharging opening 102c sensing that treatment target liquid 50 is discharged from discharging opening 102c. Alternatively, treatment target liquid 50 may be packed in microchannel 202 by: previously obtaining the volume of liquid 50 that can be packed in introducing channel 101, microchannel 202, and discharging channel 102; introducing liquid 50 of the obtained volume from introducing channel 101; and thereafter closing introduction-side valve 51 and discharge-side valve 52 by valve controller 104. Further, valve controller 104 may exert control to open or close introduction-side and discharge-side valves 51, 52 referring to the standard defining the volume of introduced liquid 50 and the period from when the liquid introduction is started until when introduction-side and discharge-side valves 51, 52 are closed, based on information including the viscosity of liquid 50, the introduction amount of liquid 50, and the capacity of introducing channel 101, microchannel 202, and discharging channel 102. Note that, when liquid 50 is introduced using a pump, liquid 50 may be packed in introducing channel 101, microchannel 202, and discharging channel 102 by the pump driven by the count with margin relative to the count obtained by: the capacity of introducing channel 101, microchannel 202, and discharging channel 102/pump discharge flow rate = pump discharge count.

Note that, since part of treatment target liquid 50 is discharged from discharging opening 102c at the downstream end of discharging channel 102, the air in introducing channel 101 and microchannel 202 before introduction of liquid 50 can be exhausted from introducing channel 101, microchannel 202, and discharging channel 102.

Here, in the case where valve controller 104 is structured by first projection 104a and second projection 104b, introduction-side and discharge-side valves 51, 52 can be controlled to close by causing housing 20a having valve controller 104 to be placed so as to be in contact with the upper surface of second substrate layer 200.

Further, in the case where valve controller 104 is structured by first and second actuators 104c, 104d and actuator controller 104e, introduction-side and discharge-side valves 51, 52 can be positioned at close position II by actuator controller 104e driving first and second actuators 104c, 104d.

### (Step S003)

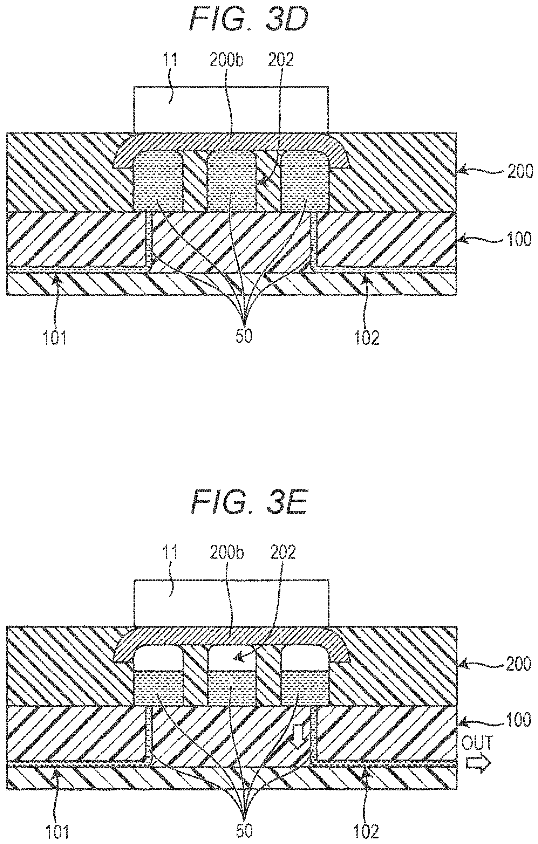

Next, as shown in FIG. 3D, heating/cooling controller 13 refers to PCR process information such as a predetermined standard, and exerts control using power source 12 to raise or lower the temperature of temperature regulator 11 that is placed so as to be in contact with metal film 200b at second substrate layer 200. The heat of temperature regulator 11 is conducted to treatment target liquid 50 in microchannel 202 via the upper surface of second substrate layer 200 (i.e., metal film 200b). Thus, a PCR process is executed on treatment target liquid 50 in microchannel 202. For example, an exemplary PCR process repeats, for 30 cycles, a series of processes, namely, an annealing process at 95°C for 30 seconds, a denaturation process at 72°C for 30 seconds, and an extension process at 60°C for 30 seconds.

### (Step S004)

After execution of the PCR process, as shown in FIG. 3E, valve controller 104 exerts control to open introduction-side and discharge-side valves 51, 52, to be positioned at open position I. Thereafter, from introducing opening 101c at first substrate layer 100, pure water or gas such as air is supplied into introducing channel 101, to discharge liquid 50 from introducing channel 101, microchannel 202, and discharging channel 102. Thus, a series of processes ends.

According to the present exemplary embodiment, the upper surface of microchannel 202 that retains treatment target liquid 50 is structured by metal film 200b, which is greater in thermal conductivity than second substrate layer 200 excluding the upper surface, and metal film 200b and liquid 50 are allowed to be in direct contact with each other. This makes it possible to reduce diffusion of heat of temperature regulator 11 via metal film 200b, and to improve responsiveness of the temperature of treatment target liquid 50 by temperature regulator 11.

Note that, the present disclosure is not limited to the present exemplary embodiment, and can be practiced in various modes.

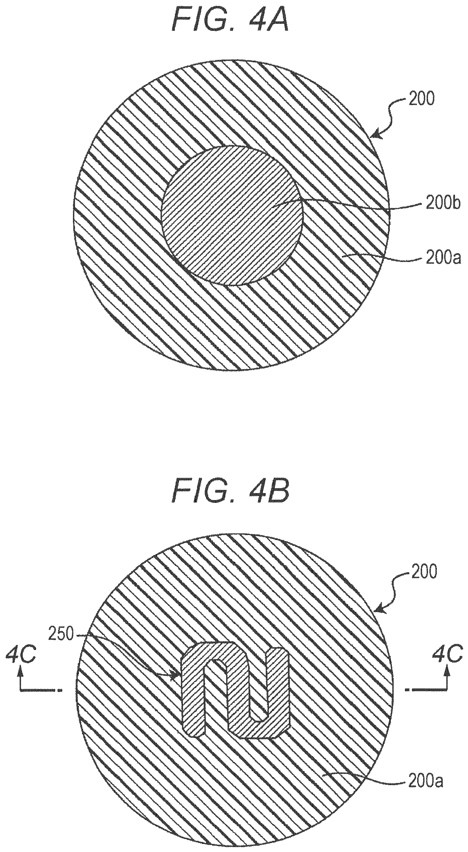

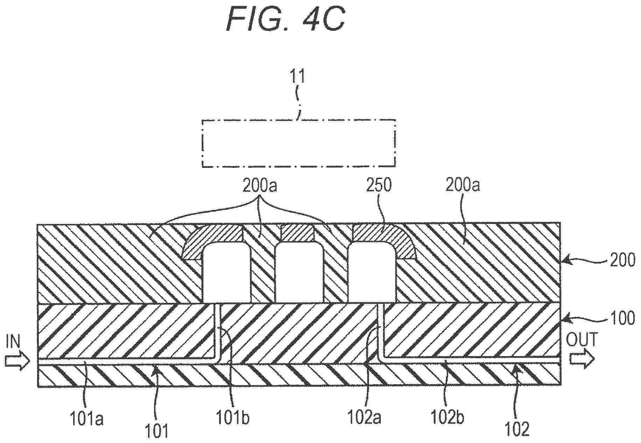

For example, in the exemplary embodiment, it has been described that metal film 200b is circular as shown in FIG. 4A. However, the present disclosure is not limited thereto, and metal film 200b may conform to the shape of microchannel 202 as shown in FIGS. 4B and 4C. For example, in FIGS. 4B and 4C, since microchannel 202 is S-shaped, metal film 250 is also S-shaped. In this manner, any portion of metal film 250 not in contact with liquid 50 can be eliminated, and heat from temperature regulator 11 can be more efficiently conducted to liquid 50.

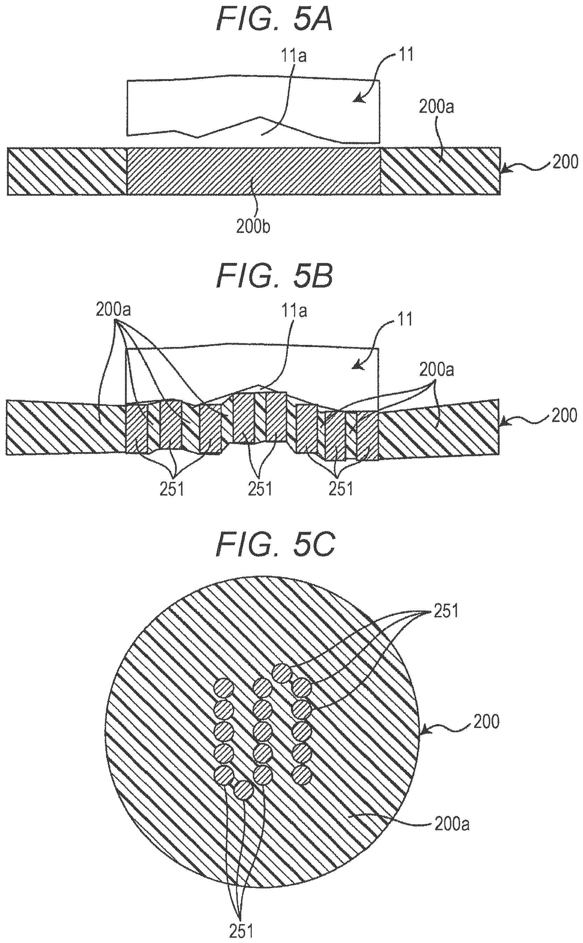

Further, metal film 200b, 250 is not limited to a single-film structure as shown in FIG. 5A. Instead, as shown in FIGS. 5B and 5C, the metal film may be a plurality of small circular metal films 251 that are arranged spaced apart from each other along S-shaped microchannel 202. It goes without saying that the present disclosure is not limited to the S-shaped arrangement, and a multitude of small circular metal films may be provided in a circular shape as shown in FIGS. 4B and 4C. In this case, since substrate layer body 200a of synthetic resin-made second substrate layer 200 exists between dotty metal films 251, the elastic force of substrate layer body 200a allows the surface where metal films 251 are arranged to become uneven so as to conform to unevenness 11a of the surface of the Peltier element, which is exemplary temperature regulator 11. Hence, metal films 251 can be closely in contact with unevenness 11a of the surface of the Peltier element. Metal films 251 are not limited to be dotted in a circular shape, and may be in any shape such as elliptical.

In this manner, since metal films 251 are brought into contact with the surface of the Peltier element with pressure by the elastic force of substrate layer body 200a, a clearance attributed to unevenness 11a of Peltier element 11 relative to metal films 251 is eliminated. Thus, the thermal conductivity between Peltier element 11 and metal films 251 can be improved. In such a case where metal films 251 are pressed by the lower surface of the Peltier element, substrate layer body 200a can serve as a buffer for metal films 251 to be efficiently brought into contact.

Further, a specific structure of metal film 200b in microchannel 202 for preventing leakage of the liquid may be as follows.

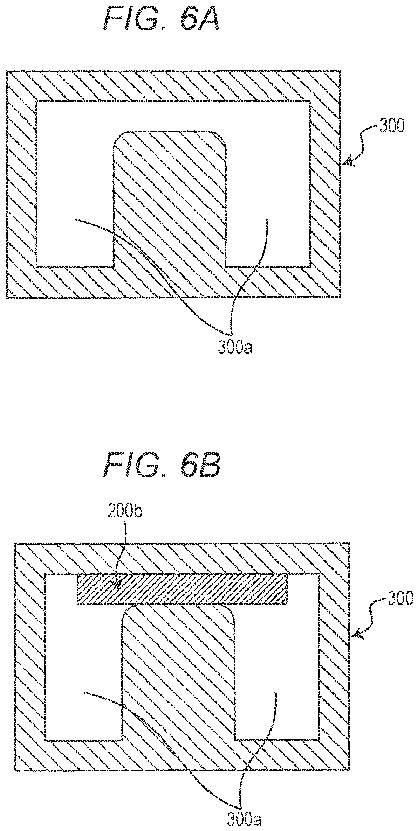

FIGS. 6A to 6E are vertical cross-sectional views for describing an exemplary process of molding microchannel 202 and metal film 200b. For the sake of clarity, the figures show the case where a portion of microchannel 202 and metal film 200b are integrally molded. In the case where the upper surface of microchannel 202 is structured by metal film 200b, it is important to prevent leakage of liquid 50 from between substrate layer body 200a and metal film 200b. To this end, as an example, when substrate layer body 200a and metal film 200b are integrally fixed in the following molding process, liquid 50 can be prevented from leaking.

Firstly, FIG. 6A shows part of mold assembly 300 for second substrate layer 200 for molding a portion of microchannel 202. Mold assembly 300 has approximately C-shaped cavity 300a for forming microchannel 202.

Next, FIG. 6B shows the state where metal film 200b is disposed at the upper surface in cavity 300a.

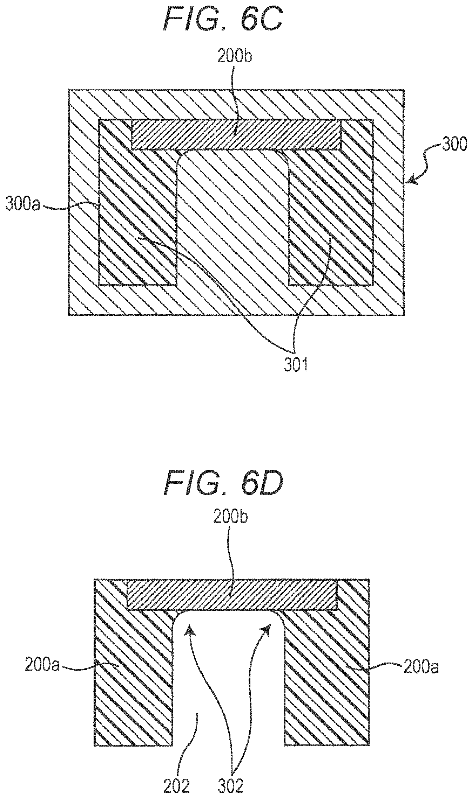

Next, FIG. 6C shows the state where synthetic resin 301, e.g., PDMS, for substrate layer body 200a of second substrate layer 200 is packed by injection molding into cavity 300a shown in FIG. 6B.

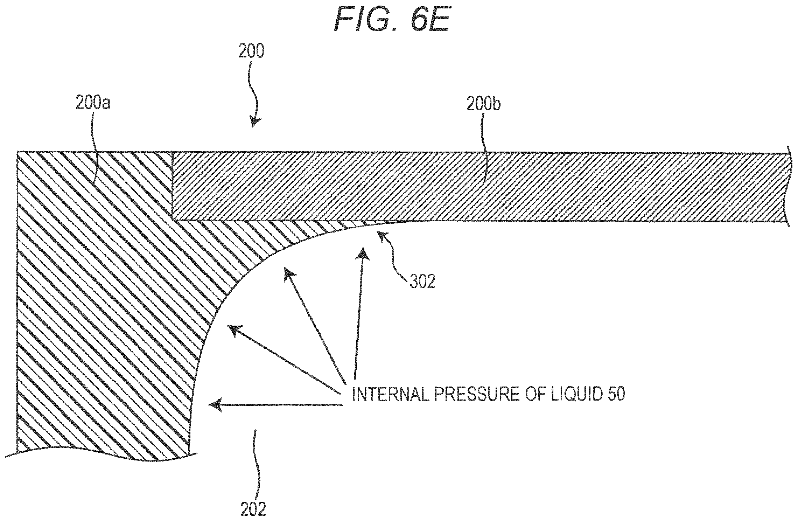

Next, FIG. 6D shows the state where a mold product of a "PCR component" is taken out from cavity 300a. The mold product is made up of substrate layer body 200a and metal film 200b integrally fixed to each other. At the corners of the upper surface of the portion of microchannel 202 in this mold product, tails 302 of synthetic resin are formed. That is, metal film 200b made of high-thermal-conductivity metal such as aluminum is previously placed in cavity 300a, and thereafter synthetic resin 301 such as PDMS is injected into cavity 300a, to form the "PCR component" of a portion of microchannel 202. In this manner, with the "PCR component", at the corners where PDMS of substrate layer body 200a are in contact with the metal of metal film 200b, smooth tails 302 are formed. Thus, the ends of metal film 200b are buried in substrate layer body 200a. As a result, tails 302 of PDMS achieve the effect of preventing leakage of liquid 50. FIG. 6E is an enlarged vertical cross-sectional view of tail 302. When liquid 50 is packed in microchannel 202 and the internal pressure of liquid 50 is applied to each tail 302, tail 302 is pressed against metal film 200b, whereby leakage of liquid 50 from between tail 302 and metal film 200b, in other words, leakage of liquid 50 from between substrate layer body 200a and metal film 200b, can be effectively prevented.

Further, though a Peltier element is used as exemplary temperature regulator 11 in the exemplary embodiment, a heat exchanger that causes hot water and cold water to flow to heat or cool the liquid may be used in place of the Peltier element.

Further, in the exemplary embodiment where Peltier element 11 and valve controller 104 for valves 51, 52 are fixed to housing 20a of heating/cooling control apparatus 20, the disposition of Peltier element 11 so as to be in contact with metal film 200b and a driving operation of valves 51, 52 are associated with each other. On the other hand, in the exemplary embodiment, by allowing Peltier element 11 or valve controller 104 to be movable relative to housing 20a, valves 51, 52 can be raised or lowered without being associated with the disposition.

Note that, any appropriate combination of the various exemplary embodiments and Variations can achieve their respective effects. Further, a combination of exemplary embodiments, a combination of Examples, or a combination of an exemplary embodiment and Example is also effective. Further, a combination of characteristics in different exemplary embodiments or Examples is also effective.

The microchannel chip, the PCR method, and the heating/cooling control apparatus according to the present disclosure reduces diffusion of heat of the temperature regulator, and the temperature regulator is capable of improving responsiveness of the temperature of the treatment target liquid. Accordingly, they are useful as a microchannel chip a PCR method, and a heating/cooling control apparatus with each of which a PCR method for sensing a DNA is performed.

### REFERENCE SINGS LIST

- 1: microchannel system (namely, microchannel apparatus)
- 10: microchannel chip
- 11: temperature regulator
- 11a: unevenness of surface of Peltier element
- 12: power source
- 13: heating/cooling controller
- 20: heating/cooling control apparatus
- 20a: housing
- 50: treatment target liquid
- 51: introduction-side valve
- 51a: valve body
- 51b: supporter
- 51c: recess
- 52: discharge-side valve
- 52a: valve body
- 52b: supporter
- 52c: recess
- 100: first substrate layer
- 100a: first substrate layer body
- 100b: back plate
- 101: introducing channel
- 101a: first channel
- 101b: second channel
- 101c: introducing opening
- 101d: fifth channel
- 101e: a pair of upward channels
- 102: discharging channel
- 102a: third channel
- 102b: fourth channel
- 102c: discharging opening
- 102d: sixth channel
- 102e: a pair of upward channels
- 104: valve controller
- 104a: first projection
- 104b: second projection
- 104c: first actuator
- 104d: second actuator
- 104e: actuator controller
- 200: second substrate layer
- 200a: substrate layer body
- 200b: metal film
- 200e: screw hole for fixing
- 202: microchannel
- 202a: first end
- 202b: second end
- 250: S-shaped metal film
- 251: a plurality of small circular metal films
- 300: mold assembly
- 300a: cavity
- 301: synthetic resin for substrate layer body
- 302: tail

## Claims

1. A polymerase chain reaction (PCR) method for performing a PCR process on a treatment target liquid (50) using a microchannel apparatus (1) including a microchannel chip (10) and a heating/cooling control apparatus (20), the PCR method comprising:
(a) preparing the microchannel apparatus (1) including the microchannel chip (10) and the heating/cooling control apparatus (20);
the microchannel chip (10) including:
a first substrate layer (100) comprising an introducing channel (101) and a discharging channel (102); and
a second substrate layer (200) that is disposed on the first substrate layer (100) and comprises a second substrate layer body (200a) and a metal film (200b), the second substrate layer body (200a) comprising a microchannel (202) connected to the introducing channel (101) and the discharging channel (102), the microchannel (202) being filled with the treatment target liquid (50), the metal film (200b) structuring an upper surface of the microchannel (202),
the heating/cooling control apparatus (20) including:
a temperature regulator (11) that is disposed so as to be in contact with the metal film (200b) of the second substrate layer (200) and is capable of heating or cooling the metal film (200b);
a power source (12) that applies voltage to the temperature regulator (11); and
a heating/cooling controller (13) that controls the voltage applied from the power source (12) to the temperature regulator (11);
(b) controlling, with the heating/cooling controller (13), the voltage applied from the power source (12) to the temperature regulator (11) so as to change a temperature of the treatment target liquid (50) in the microchannel (202), thereby achieving the PCR process,
wherein an end of the metal film (200b) of the second substrate layer (200) in the microchannel chip is buried in the second substrate layer body (200a).

2. The PCR method according to claim 1, wherein
the microchannel chip (10) further includes:
an introduction-side valve (51) capable of opening or closing the introducing channel (101); and
a discharge-side valve (52) capable of opening or closing the discharging channel (102),
the heating/cooling control apparatus (20) further includes:
a valve controller that controls the introduction-side valve (51) and the discharge-side valve (52) to open or close,
the PCR method further comprising, prior to the step (b),
(c) closing the introduction-side valve (51) and the discharge-side valve (52) under control of the valve controller in a state where the microchannel (202) is filled with the treatment target liquid (50).

3. The PCR method according to claim 1, wherein the second substrate layer body (200a) is made of dimethylpolysiloxane, and the metal film (200b) is made of aluminum.

4. The PCR method according to claim 1, wherein the temperature regulator (11) is a Peltier element.

5. The PCR method according to claim 1, wherein the metal film (200b) includes a plurality of metal films that are spaced apart from each other and arranged along the microchannel (202).

6. The PCR method according to claim 2, further comprising, after the step (b),
(d) opening the introduction-side valve (51) and the discharge-side valve (52) so as to discharge, from the discharge-side valve (52), the treatment target liquid (50) having undergone the PCR process.

7. The PCR method according to claim 1, wherein, in the step (b), the heating/cooling control apparatus (20) controls the change in the temperature of the temperature regulator (11) in accordance with a type of the treatment target liquid (50), based on relationship information among the type of the treatment target liquid (50), the voltage to the temperature regulator (11), a period of applying the voltage, and the temperature.

8. A microchannel apparatus used for performing a polymerase chain reaction (PCR) process on a treatment target liquid (50), the microchannel apparatus including a microchannel chip (10) and a heating/cooling control apparatus (20),
the microchannel chip (10) comprising:
a first substrate layer (100) that comprises an introducing channel (101) and a discharging channel (102); and
a second substrate layer (200) that is disposed on the first substrate layer (100) and comprises a second substrate layer body (200a) and a metal film (200b),
wherein the second substrate layer body (200a) is connected to the introducing channel (101) and the discharging channel (102), and comprises a microchannel (202) for being filled with the treatment target liquid (50),
the metal film (200b) structures an upper surface of the microchannel (202), and
the heating/cooling control apparatus (20) including:
a temperature regulator (11) that is disposed so as to be in contact with the metal film (200b) of the second substrate layer (200) and is capable of heating or cooling the metal film (200b);
a power source (12) that applies voltage to the temperature regulator (11); and
a heating/cooling controller (13) that controls the voltage applied from the power source (12) to the temperature regulator (11);
in a state where the temperature regulator (11) is in contact with the metal film (200b), the heating/cooling controller (13) is configured to control the voltage applied from the power source (12) to the temperature regulator (11) so as to change a temperature of the treatment target liquid (50) having been introduced from the introducing channel (101) so that the microchannel (202) is filled with the treatment target liquid (50), thereby achieving the PCR process,
wherein an end of the metal film (200b) of the second substrate layer (200) in the microchannel chip is buried in the second substrate layer body (200a).

9. The microchannel apparatus according to claim 8, wherein
the microchannel chip (10) further includes:
an introduction-side valve (51) that is capable of opening or closing the introducing channel (101); and
a discharge-side valve (52) that is capable of opening or closing the discharging channel (102).

10. The microchannel appartaus according to claim 8, wherein the second substrate layer body (200a) is made of dimethylpolysiloxane, and the metal film (200b) is made of aluminum.

## Patentansprüche

1. Polymerase-Kettenreaktions-(PCR)-Verfahren zur Durchführung eines PCR-Prozesses an einer Behandlungszielflüssigkeit (50) unter Verwendung einer Mikrokanalvorrichtung (1), die einen Mikrokanalchip (10) und eine Heiz-/ Kühlsteuervorrichtung (20) enthält, wobei das PCR-Verfahren umfasst:
(a) Vorbereiten der Mikrokanalvorrichtung (1), die den Mikrokanalchip (10) und die Heiz-/Kühlsteuervorrichtung (20) enthält; wobei
der Mikrokanalchip (10) umfasst:
eine erste Substratschicht (100) mit einem Einleitkanal (101) und einem Auslasskanal (102); und
eine zweite Substratschicht (200), die auf der ersten Substratschicht (100) angeordnet ist und einen zweiten Substratschichtaufbau (200a) und eine Metallfolie (200b) aufweist, wobei der zweite Substratschichtaufbau (200a) einen Mikrokanal (202) einschließt, der mit dem Einleitkanal (101) und dem Auslasskanal (102) verbunden ist, der Mikrokanal (202) mit der Behandlungszielflüssigkeit (50) gefüllt ist und die Metallfolie (200b) eine Oberfläche des Mikrokanals (202) strukturiert,
die Heiz-/Kühlsteuervorrichtung (20) enthält:
einen Temperaturregler (11), der so angeordnet ist, dass er sich mit der Metallfolie (200b) der zweiten Substratschicht (200) in Kontakt befindet und in der Lage ist, die Metallfolie (200b) zu erhitzen oder zu kühlen;
eine Stromquelle (12), die an den Temperaturregler (11) Spannung anlegt; und
eine Heiz-/Kühlsteuereinheit (13), welche die von der Stromquelle (12) an den Temperaturregler (11) angelegte Spannung steuert;
(b) Steuern, mit der Heiz-/Kühlsteuereinheit (13), die von der Stromquelle (12) an den Temperaturregler (11) angelegte Spannung, um eine Temperatur der Behandlungszielflüssigkeit (50) in dem Mikrokanal (202) zu ändern, wodurch der PCR-Prozess erreicht wird,
wobei ein Ende der Metallfolie (200b) der zweiten Substratschicht (200) im Mikrokanalchip in dem zweiten Substratschichtaufbau (200a) vergraben wird.

2. PCR-Verfahren nach Anspruch 1, wobei
der Mikrokanalchip (10) des Weiteren enthält:
ein einleitungsseitiges Ventil (51), das in der Lage ist, den Einleitkanal (101) zu öffnen oder zu schließen; und
ein auslassseitiges Ventil (52), das in der Lage ist, den Auslasskanal (102) zu öffnen oder zu schließen,
wobei die Heiz-/Kühlsteuervorrichtung (20) des Weiteren enthält:
eine Ventilsteuereinheit, die das einleitungsseitige Ventil (51) und das auslassseitige Ventil (52) zum Öffnen oder Schließen steuert,
wobei das PCR-Verfahren des Weiteren vor dem Schritt (b)
(c) Schließen des einleitungsseitigen Ventils (51) und des auslassseitigen Ventils (52) unter Steuerung der Ventilsteuereinheit in einem Zustand, bei dem der Mikrokanal (202) mit der Behandlungszielflüssigkeit (50) gefüllt ist, umfasst.

3. PCR-Verfahren nach Anspruch 1, wobei der zweite Substratschichtaufbau (200a) aus Dimethylpolysiloxan besteht und die Metallfolie (200b) aus Aluminium besteht.

4. PCR-Verfahren nach Anspruch 1, wobei der Temperaturregler (11) ein Peltier-Element ist.

5. PCR-Verfahren nach Anspruch 1, wobei die Metallfolie (200b) eine Vielzahl von Metallfolien enthält, die im Abstand voneinander und längs des Mikrokanals (202) angeordnet sind.

6. PCR-Verfahren nach Anspruch 2, des Weiteren umfassend, nach dem Schritt (b),
(d) Öffnen des einleitungsseitigen Ventils (51) und des auslassseitigen Ventils (52), um aus dem auslassseitigen Ventil (52) die Behandlungszielflüssigkeit (50), nachdem sie den PCR-Prozess durchgemacht hat, zu entleeren.

7. PCR-Verfahren nach Anspruch 1, wobei im Schritt (b) die Heiz-/Kühlsteuervorrichtung (20) die Temperaturänderung des Temperaturreglers (11) entsprechend einem Typ der Behandlungszielflüssigkeit (50) steuert, basierend auf Beziehungsinformationen zwischen dem Typ der Behandlungszielflüssigkeit (50), der Spannung an den Temperaturregler (11), einer Zeitdauer zum Anlegen der Spannung und der Temperatur.

8. Mikrokanalvorrichtung, die zur Durchführung eines Polymerase-Kettenreaktions-(PCR)-Prozesses an einer Behandlungszielflüssigkeit (50) verwendet wird, wobei die Mikrokanalvorrichtung einen Mikrokanalchip (10) und eine Heiz-/Kühlsteuervorrichtung (20) enthält,
wobei der Mikrokanalchip (10) umfasst:
eine erste Substratschicht (100), die einen Einleitkanal (101) und einen Auslasskanal (102) enthält; und
eine zweite Substratschicht (200), die auf der ersten Substratschicht (100) angeordnet ist und einen zweiten Substratschichtaufbau (200a) und eine Metallfolie (200b) einschließt,
wobei der zweite Substratschichtaufbau (200a) mit dem Einleitkanal (101) und dem Auslasskanal (102) verbunden ist und einen Mikrokanal (202) enthält, um mit der Behandlungszielflüssigkeit (50) gefüllt zu werden,
die Metallfolie (200b) eine obere Fläche des Mikrokanals (202) strukturiert, und
die Heiz-/Kühlsteuervorrichtung (20) umfasst:
einen Temperaturregler (11), der so angeordnet ist, dass er sich mit der Metallfolie (200b) der zweiten Substratschicht (200) in Kontakt befindet und in der Lage ist, die Metallfolie (200b) zu erhitzen oder zu kühlen;
eine Stromquelle (12), die an den Temperaturregler (11) Spannung anlegt; und eine Heiz-/Kühlsteuereinheit (13), welche die von der Stromquelle (12) an den Temperaturregler (11) angelegte Spannung steuert;
in einem Zustand, bei dem der Temperaturregler (11) sich mit der Metallfolie (200b) in Kontakt befindet, wobei die Heiz-/Kühlsteuereinheit (13) ausgelegt ist, die von der Stromquelle (12) an den Temperaturregler (11) angelegte Spannung zu steuern, um eine Temperatur der Behandlungszielflüssigkeit (50) zu ändern, die vom Einleitkanal (101) eingeleitet wurde, so dass der Mikrokanal (202) mit der Behandlungszielflüssigkeit (50) gefüllt ist, wodurch der PCR-Prozess erreicht wird,
wobei ein Ende der Metallfolie (200b) der zweiten Substratschicht (200) im Mikrokanalchip in dem zweiten Substratschichtaufbau (200a) vergraben wird.

9. Mikrokanalvorrichtung nach Anspruch 8, wobei
der Mikrokanalchip (10) des Weiteren enthält:
ein einleitungsseitiges Ventil (51), das in der Lage ist, den Einleitkanal (101) zu öffnen oder zu schließen; und
ein auslassseitiges Ventil (52), das in der Lage ist, den Auslasskanal (102) zu öffnen oder zu schließen.

10. Mikrokanalvorrichtung nach Anspruch 8, wobei der zweite Substratschichtaufbau (200a) aus Dimethylpolysiloxan und die Metallfolie (200b) aus Aluminium besteht.

## Revendications

1. Procédé de réaction en chaîne par polymérase (PCR) pour effectuer un processus PCR sur un liquide cible de traitement (50) en utilisant un appareil à microcanal (1) comprenant une puce à microcanal (10) et un appareil de commande de chauffage/ refroidissement (20), le procédé PCR consistant à:
(a) préparer l'appareil à microcanal (1) comprenant la puce à microcanal (10) et l'appareil de commande de chauffage/refroidissement (20);
la puce à microcanal (10) comprenant:
une première couche de substrat (100) comprenant un canal d'intro-duction (101) et un canal de décharge (102); et
une seconde couche de substrat (200) qui est disposée sur la première couche de substrat (100) et comprend un second corps de couche de substrat (200a) et un film métallique (200b), le second corps de couche de substrat (200a) comprenant un microcanal (202) relié au canal d'introduction (101) et le canal de décharge (102), le microcanal (202) étant rempli du liquide cible de traitement (50), le film métallique (200b) structurant une surface supérieure du microcanal (202),
l'appareil de commande de chauffage/refroidissement (20) comprenant:
un régulateur de température (11) qui est disposé de manière à être en contact avec le film métallique (200b) de la seconde couche de substrat (200) et est susceptible de chauffer ou de refroidir le film métallique (200b);
une source d'énergie (12) qui applique une tension au régulateur de température (11); et
un contrôleur de chauffage/refroidissement (13) qui commande la tension appliquée par la source de puissance (12) au régulateur de température (11);
(b) commander, avec le contrôleur de chauffage/refroidissement (13), la tension appliquée à partir d'une source d'énergie (12) au régulateur de température (11) de manière à changer la température du liquide cible de traitement (50) dans le microcanal (202), réalisant ainsi la PCR processus, dans lequel une extrémité du film métallique (200b) de la seconde couche de substrat (200) dans la puce de micro canal est enfouie dans le second corps de couche de substrat (200a).

2. Procédé de PCR selon la revendication 1, dans lequel
la puce à microcanal (10) comprend en outre:
une valve côté introduction (51) susceptible d'ouvrir ou de fermer le canal d'introduction (101); et
une valve côté décharge (52) susceptible d'ouvrir ou de fermer le canal de décharge (102),
l'appareil de commande de chauffage/refroidissement (20) comprend en outre:
un contrôleur de valve qui commande l'ouverture ou la fermeture de la valve côté introduction (51) et de la valve côté décharge (52),
le procédé de PCR consistant en outre, avant l'étape (b), à
(c) fermer la valve côté introduction (51) et la valve côté décharge (52) sous la commande du contrôleur de valve lorsque le microcanal (202) est rempli du liquide cible de traitement (50).

3. Procédé de PCR selon la revendication 1, dans lequel le second corps de couche de substrat (200a) est constitué de diméthylpolysiloxane, et le film métallique (200b) est constitué d'aluminium.

4. Procédé de PCR selon la revendication 1, dans lequel le régulateur de température (11) est un Élément Peltier.

5. Procédé de PCR selon la revendication 1, dans lequel le film métallique (200b) comprend une pluralité de films métalliques qui sont espacés l'un de l'autre et disposés le long du microcanal (202).

6. Procédé PCR selon la revendication 2, consistant en outre, après l'étape (b), à:
(d) ouvrir la valve côté introduction (51) et la valve côté décharge (52) de façon à décharger, à partir de la valve côté décharge (52), le liquide cible de traitement (50) ayant subi le processus de PCR.

7. Procédé de PCR selon la revendication 1, dans lequel, à l'étape (b), l'appareil de commande de chauffage/refroidissement (20) contrôle le changement de température du régulateur de température (11) selon un type de liquide cible de traitement (50), sur la base des informations de relation parmi le type du liquide cible de traitement (50), la tension au régulateur de température (11), une période d'application de la tension et la température.

8. Appareil à microcanal permettant de réaliser un procédé de réaction en chaîne par polymérase (PCR) sur un liquide cible de traitement (50), l'appareil à microcanal comprenant une puce à microcanal (10) et un appareil de commande de chauffage/ refroidissement (20),
la puce à microcanal (10) comprenant:
une première couche de substrat (100) comprenant un canal d'intro-duction (101) et un canal de décharge (102); et
une seconde couche de substrat (200) qui est disposée sur la première couche de substrat (100) et comprend un second corps de couche de substrat (200a) et un film métallique (200b),
dans lequel le second corps de couche de substrat (200a) est connecté au canal d'introduction (101) et au canal de décharge (102), et comprend un microcanal (202) devant être rempli du liquide cible de traitement (50),
le film métallique (200b) structures une surface supérieure du microcanal (202),
et l'appareil de commande de chauffage/refroidissement (20) comprenant:
un régulateur de température (11) qui est disposé de manière à être en contact avec le film métallique (200b) de la seconde couche de substrat (200) et est susceptible de chauffer ou de refroidir le film métallique (200b); une source d'énergie (12) qui applique une tension au régulateur de température (11); et
un contrôleur de chauffage/refroidissement (13) qui commande la tension appliquée par la source de puissance (12) au régulateur de température (11);
lorsque le régulateur de température (11) est en contact avec le film métallique (200b), le contrôleur de chauffage/refroidissement (13) est configuré pour commander la tension appliquée à partir de la source d'alimentation (12) au régulateur de température (11) de manière à modifier la température du liquide cible de traitement (50) ayant été introduit à partir du canal d'introduction (101) de sorte que le microcanal (202) est rempli du liquide cible de traitement (50), réalisant ainsi le procédé PCR,
dans lequel une extrémité du film métallique (200b) de la seconde couche de substrat (200) dans la puce de micro canal est enfouie dans le second corps de couche de substrat (200a).

9. Procédé de PCR selon la revendication 8, dans lequel
la puce à microcanal (10) comprend en outre:
une valve côté introduction (51) susceptible d'ouvrir ou de fermer le canal d'introduction (101); et
une valve côté décharge (52) qui est capable d'ouvrir ou de fermer le canal de décharge (102).

10. Appareil de PCR selon la revendication 8, dans lequel le second corps de couche de substrat (200a) est constitué de diméthylpolysiloxane, et le film métallique (200b) est constitué d'aluminium.
